# FASCICULE DE BREVET EUROPEEN

(11) **EP 1 738 094 B1**
(45) Date de publication et mention de la délivrance du brevet: **16.04.2008**
(21) Numéro de dépôt: 05742143.0
(22) Date de dépôt: 17.03.2005
(51) Int. Cl.: F16H 21/36, A61B 8/10

(54) **MECANISME D'ENTRAINEMENT UTILISABLE DANS UN DISPOSITIF DE BALAYAGE**
IN EINER ABTASTVORRICHTUNG VERWENDBARER ANTRIEBSMECHANISMUS
DRIVE MECHANISM WHICH CAN BE USED IN A SCANNING DEVICE

(30) Priorité: 01.04.2004 FR 0403523
(43) Date de publication de la demande: 03.01.2007
(73) Titulaire: Quantel Medical, 63100 Clermond Ferrand (FR)
(72) Inventeur: PETETIN, Arnaud, F-63780 Saint Georges de Mons (FR)
(74) Mandataire: de Saint-Palais, Arnaud Marie
(86) Numéro de dépôt international: PCT/FR2005/000677
(87) Numéro de publication internationale: WO 2005/098274

(56) Documents cités:
- US-A- 2 360 762
- US-A- 4 215 585
- US-A- 5 331 962
- US-A- 5 333 612
- US-A1- 2003 220 572

## Description

La présente invention concerne un mécanisme d'entraînement utilisable dans un dispositif de balayage.

Elle s'applique notamment, mais non exclusivement, à la réalisation d'une sonde échographique faisant intervenir un élément transducteur mobile selon un trajet prédéterminé par exemple rectiligne ou même arciforme à courbure fixe ou variable. Un mouvement sectoriel est également possible.

On sait que dans de nombreux domaines d'application, on est amené à utiliser des sondes échographiques présentant des dimensions de plus en plus réduites, ces sondes devant nécessairement faire intervenir un transducteur monté sur un mécanisme plus ou moins complexe entraîné le plus souvent par un moto-réducteur électrique. Ces sondes présentent le plus souvent un corps tubulaire, de forme sensiblement cylindrique, dont le diamètre intérieur correspond sensiblement au diamètre du moto-réducteur. Le mécanisme d'entraînement du transducteur doit alors rentrer dans un volume cylindrique dont le diamètre est aussi voisin que possible, voire inférieur à celui du moteur. Du fait de la miniaturisation des moteurs et des exigences imposées par le mode d'application de la sonde, le volume dédié à ces mécanismes devient de plus en plus petit. Or, ces mécanismes présentent souvent des cinématiques relativement complexes. Leur réalisation devient alors mal aisée, voire même problématique.

Par ailleurs, le niveau de précision requis par ces mécanismes ainsi que par les capteurs qui leurs sont associés à des fins d'asservissement est habituellement très élevé. Ici également, la réduction du volume disponible tend à augmenter la difficulté que l'on rencontre pour atteindre de tels niveaux de précision.

Ainsi, par exemple, ces mécanismes font intervenir généralement des cinématiques permettant de convertir le mouvement de rotation du moteur en un mouvement rectiligne alternatif utilisable pour l'entraînement de l'élément transducteur. Néanmoins, la plupart de ces dispositifs tels que, par exemple, le système bielle/manivelle, présentent un encombrement relativement important et ne conviennent donc pas bien dans le cas où le mouvement rectiligne doit s'effectuer dans un plan radial de la sonde.

De plus, la miniaturisation des systèmes donne de moins en moins de place aux éléments d'articulation, tels que paliers ou roulements, utilisés dans de tels systèmes.

Le document US 5,333,612 A divulgue un mécanisme d'entraînement tel que décrit dans le préambule de la revendication 1.

L'invention a donc plus particulièrement pour but de supprimer ces inconvénients.

A cet effet, elle propose un mécanisme d'entraînement faisant intervenir un plateau tournant planétaire entraîné en rotation par l'arbre de sortie d'une motorisation, un pignon satellite monté pivotant sur le plateau et venant engrener avec une couronne à alésage dentelé coaxiale audit arbre et solidaire du corps du moteur et un organe d'entraînement axial porté par un support solidaire du pignon, le diamètre du pignon étant égal à la moitié du diamètre de l'alésage de la couronne et l'organe d'entraînement étant disposé de manière à ce que, lors de la rotation du plateau, ledit organe effectue une trajectoire rectiligne reliant deux points diamétralement opposés de la couronne.

Grâce à ces dispositions, on obtient un mécanisme d'entraînement occupant un volume cylindrique plat coaxial au moteur et sensiblement de même diamètre. Le mouvement de l'organe d'entraînement, de type sinusoïdal, est obtenu avec un frottement minimum, avec une faible usure et un jeu très réduit.

On constate que ce mécanisme se prête parfaitement bien à un système asservi.

Avantageusement, dans le cas où ce mécanisme est utilisé dans une sonde pour déplacer l'élément transducteur selon un trajet linéaire, l'organe d'entraînement pourra être couplé à une pièce de support du transducteur, cette pièce de support pouvant être montée coulissante le long d'une glissière solidaire du corps de la sonde. Ce couplage pourra s'effectuer soit en prise directe, soit à distance, par exemple grâce à un couplage magnétique.

De même, ce mécanisme pourra être intégré dans une chaîne cinématique plus complexe permettant par exemple de convertir un mouvement rectiligne alternatif en un mouvement arciforme.

La présente invention, sur la base du mécanisme de balayage présenté ci-dessus et décrit ci-après, permet, en rajoutant un étage de balayage, d'obtenir n'importe quel mouvement, permettant par exemple de guider un élément piézoélectrique simplement, tout en se mettant dans les meilleures conditions possibles pour obtenir une image.

Des modes d'exécution de l'invention seront décrits ci-après, à titre d'exemples non limitatifs, avec référence aux dessins annexés dans lesquels :
Les figures 1 et 2 sont des coupes axiales de deux variantes d'exécution d'une sonde échographique à mouvement linéaire ;
Les figures 3 et 4 sont des coupes axiales à 90° l'une de l'autre d'une sonde échographique à mouvement arciforme.

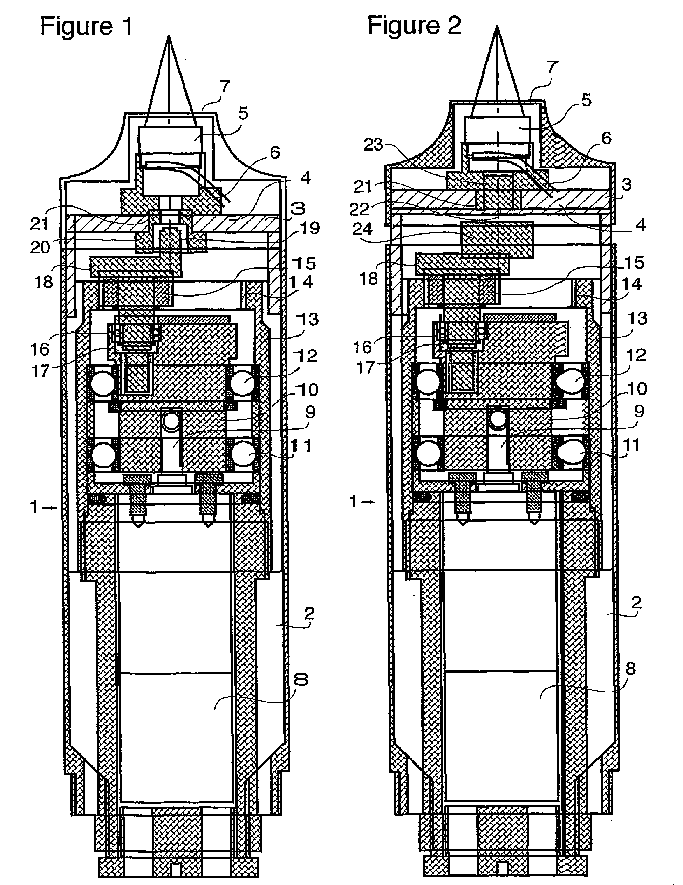

Dans les exemples représentés figures 1 et 2, la sonde comprend un corps tubulaire 1 divisé en deux compartiments 2, 3 par une cloison transversale 4. Le compartiment antérieur 3 loge un élément transducteur 5 monté sur une pièce support 6 mobile en translation sur la cloison 4. Ce transducteur 5 est conçu de manière à émettre un rayonnement ultrasonore focalisé au travers de la paroi antérieure 7 de la sonde.

Dans l'exemple représenté sur la figure 2, ce compartiment antérieur 3 est étanche et peut être rempli par un liquide permettant d'assurer une bonne transmission des ondes ultrasonores.

Le compartiment postérieur 2 renferme un moto-réducteur ainsi qu'un mécanisme de conversion du mouvement rotatif de l'arbre de sortie 9 de ce moteur 8 en un mouvement alternatif rectiligne.

Ce mécanisme fait intervenir une pièce d'entraînement cylindrique 10 monté rotative coaxialement à l'arbre de sortie 9 du moteur 8 par l'intermédiaire de deux paliers (ou roulements à billes) 11, 12 axialement décalés portés par un manchon tubulaire 13 solidaire du corps du moteur 8.

Ce manchon tubulaire 13 comprend, à son extrémité antérieure, une denture interne (couronne dentée 14) sur laquelle engrène un pignon satellite 15 monté pivotant sur la pièce d'entraînement 10 grâce à un arbre 16 qui s'engage dans un perçage cylindrique 17 de la pièce d'entraînement 10, axé parallèlement à l'axe 9 du moteur 8, à une distance prédéterminée de celui-ci. Le montage rotatif de l'arbre 16 dans le perçage 17 est assuré au moyen d'un palier (ou d'un roulement à billes) prévu entre ledit arbre 16 et la paroi dudit perçage 17.

Le pignon 15 porte par sa face supérieure une pièce de support 18 d'un organe d'entraînement de la pièce de support 6 de l'élément transducteur 5.

Dans l'exemple représenté sur la figure 1, l'organe d'entraînement consiste en une broche axiale 19 qui s'engage dans la cavité d'un coulisseau 20 mobile le long d'une fente 2 1 prévue dans la cloison 4 et qui est fixée à la pièce de support 6.

Ainsi, lors de la rotation du moteur 8, le pignon 15 porté par la pièce d'entraînement 18 tourne selon un trajet circulaire coaxial. Le long de ce trajet, il engrène sur la denture 14 du manchon tubulaire 13 en tournant sur lui-même autour d'un axe parallèle à l'arbre 9 du moteur 8.

Le mouvement de la broche 19 qui correspond au produit de la double rotation (planétaire/satellite) est un mouvement alternatif rectiligne. La cloison 4 est agencée de manière à ce que le trajet de la broche suive le trajet de la fente 21 et qu'ainsi, l'élément transducteur effectue lui-même un déplacement alternatif rectiligne.

Avantageusement, la cavité du coulisseau 20 destinée à accueillir la broche 19 sera oblongue, de manière à tolérer des différences d'alignement.

Dans l'exemple représenté sur la figure 2, la cloison 4 comprend au lieu d'une fente, une gorge 21 refermée par un fond 22. La pièce de support 6 présente une forme en T dont la branche verticale s'engage et est guidée dans la gorge 21. Cette branche verticale dont la largeur correspond à celle de la gorge 21 comprend une cavité centrale logeant un premier aimant permanent 23.

L'organe d'entraînement consiste ici en un second aimant permanent 24 de polarité inversée par rapport au premier, fixé sur la face supérieure de la pièce de support 18. Cet aimant 24 est donc mobile selon un trajet rectiligne parallèlement et au voisinage de la cloison 4.

On obtient ainsi un couplage magnétique des deux aimants permanents 23, 24 et un entraînement sans contact de l'aimant 23 par l'aimant 24 le long de la gorge 21.

Bien entendu, l'invention ne se limite pas à une forme particulière de déplacement.

Ainsi, le mécanisme selon l'invention est utilisable à l'entraînement d'une cinématique de conversion, mouvement réctiligne/mouvement arciforme.

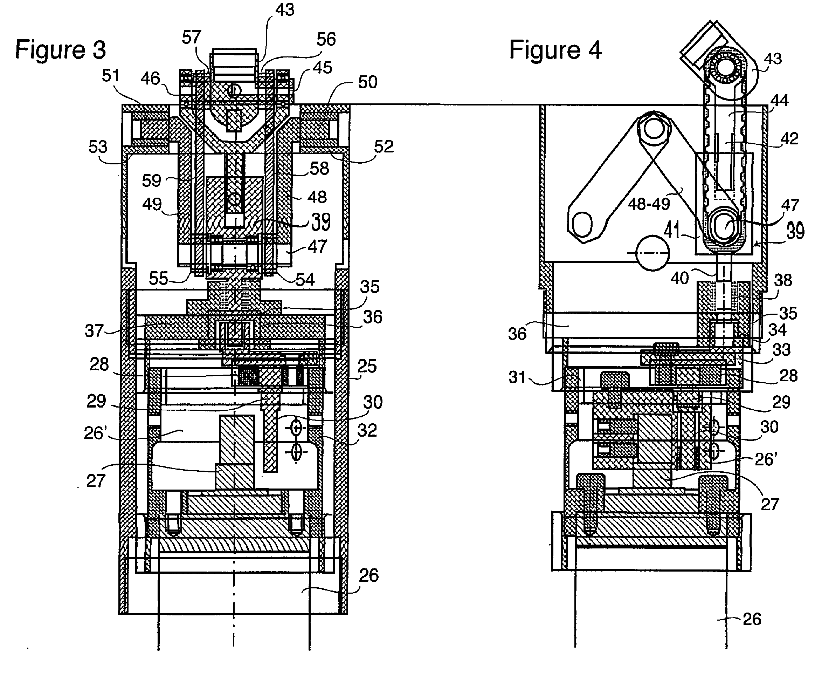

Les figures 3 et 4 illustrent un mode d'exécution d'une telle application.

Ces figures représentent une sonde d'échographie oculaire à mouvement arciforme utilisant un mécanisme d'entraînement à mouvement rectiligne similaire à celui utilisé dans la sonde représentée sur les figures 1 et 2.

On rappelle que ce type de sonde a la particularité de tenir compte du fait que la cornée n'est pas véritablement sphérique mais présente des variations importantes entre son centre et la périphérie : En fait, le plan de base de la cornée présente une forme elliptique de grand diamètre D de l'ordre de 12 mm et de petit diamètre, de l'ordre de 11 mm, la différence de diamètre provenant de l'ouverture et la fermeture des paupières.

Par ailleurs, il est admis que la cornée présente deux zones, une zone centrale qui est sphérique et une zone périphérique dans laquelle le rayon de courbure augmente progressivement vers le limbe. Il apparaît donc que la cornée est une calotte asphérique et asymétrique, qui s'aplatit progressivement vers la périphérie. Du fait des différents rayons de courbure entre la cornée et la sclère, la jonction de la cornée et de la sclère présente un sulcus apparent au niveau de l'angle irido-cornéen.

L'avantage du balayage arciforme est de permettre à la sonde de suivre une trajectoire dont le rayon de courbure est fixe et sensiblement égal au rayon de courbure moyen de la cornée, tout en maintenant l'axe du faisceau ultrasonore orthogonal à une grande partie de la surface de la cornée et/ou de la rétine, en vue d'améliorer la qualité du signal échographique reçu par la sonde tout en évitant que celle-ci ne se rapproche de la sclère en risquant de la heurter.

La sonde illustrée sur les figures 3 et 4 a donc pour but de parvenir à ces résultats, au moyen d'un mécanisme permettant de réduire considérablement les dimensions de la sonde tout en conservant des niveaux de précision et de performance élevés.

Cette sonde comprend une structure de support tubulaire 25 renfermant dans sa partie inférieure un moteur 26 dont l'arbre de sortie 27 entraîne une pièce cylindrique 26' sur laquelle est monté rotatif un pignon 28 grâce à un axe 29 qui s'engage dans un guidage composé de palier et de roulements à billes monté dans un perçage 30 réalisé dans la face antérieure de la pièce cylindrique, parallèlement à l'axe de l'arbre 27 et à une distance prédéterminée de celui-ci.

Ce pignon 28 engrène avec la denture d'une couronne dentée 31 portée par un manchon tubulaire 32 solidaire du corps du moteur 26. Il supporte ici une pièce d'entraînement 33 munie d'un doigt d'entraînement axial 34 qui, lors de la rotation du moteur 26, effectue un déplacement rectiligne le long d'un diamètre du manchon tubulaire 32.

Ce doigt 34 s'engage dans l'élément postérieur d'un coulisseau 35 tubulaire passant au travers d'une fente 36 réalisée dans une cloison transversale 37 solidaire du manchon tubulaire 32.

Ce coulisseau 35 est réalisé par assemblage de deux éléments tubulaires épaulés antérieur/postérieur, dont les épaulements viennent en retour sur la cloison 37. Ce coulisseau peut donc se déplacer le long de la fente 36 tout en étant retenu axialement dans les deux sens par les épaulements.

Le doigt 34 comprend un alésage coaxial qui s'étend dans le prolongement d'un alésage 38 de l'élément antérieur du coulisseau 35 de manière à constituer avec celui-ci un palier cylindrique.

Dans ce palier est montée axialement coulissante une pièce support cylindrique 39 dont la partie inférieure 40 s'engage dans le palier cylindrique et dont la partie supérieure 41 de plus grand diamètre sert, d'une part, de support à un bras 42 portant l'élément transducteur 43 de la sonde et, d'autre part, d'articulation à un embiellage.

D'une façon plus précise, la partie supérieure de la pièce 39 comprend un alésage coaxial dans lequel s'engage et est fixée par une clavette une tige dont l'extrémité antérieure en forme de fourche constitue une chape d'articulation 44. Cette chape 44 comprend deux perçages coaxiaux transversaux dans lesquels sont montés, sur roulements à billes, deux tourillons coaxiaux 45, 46 solidaires de l'élément transducteur 43.

Par ailleurs, la pièce 39 comprend un perçage transversal dans lequel est monté pivotant, sur roulements à billes, un axe transversal 47, dont les deux extrémités qui dépassent de la pièce sont respectivement solidaires des extrémités de deux bielles longitudinales parallèles 48, 49 constituant le susdit embiellage.

Les extrémités de ces deux bielles 48, 49, opposées à l'axe 47, sont munies de deux tourillons respectifs coaxiaux 50, 51 axés parallèlement à l'axe 47, qui s'engagent et sont montés pivotants dans deux paliers respectifs situés dans un plan axial perpendiculaire à la fente. Ces paliers sont disposés dans des logements 52, 53 prévus dans la structure tubulaire 25, au voisinage de son ouverture antérieure.

Les extrémités de l'axe 47 ressortant de la pièce 39 comportent deux poulies crantées respectives 54, 55 situées au droit de deux poulies correspondantes 56, 57 prévues sur les tourillons 45, 46 de l'élément transducteur 43.

Les couples de poulies en regard sont reliés par deux courroies crantées 58, 59 respectives.

Grâce à ces dispositions, lorsque le moteur 26 est actionné, le doigt 34 effectue un déplacement alternatif linéaire le long de la gorge 36 de la façon indiquée en regard de la figure 1.

Au cours de ce déplacement, il engendre un déplacement en translation du coulisseau 35 et un basculement des deux bielles 48, 49 autour de l'axe des tourillons 50, 51. La pièce 39 qui se trouve entraînée en translation par le coulisseau sous l'effet du déplacement circulaire de l'axe 47 effectue un déplacement axial en coulissant dans le palier de l'alésage 38.

En conséquence, l'axe des tourillons 45, 46 décrit un trajet arciforme qui est le produit du déplacement en translation engendré par le coulisseau 35 et du déplacement axial engendré par les bielles 48, 49.

Au cours de ce déplacement, grâce à l'action des courroies crantées 58, 59, l'orientation de l'élément transducteur 43 varie en fonction de l'orientation des bielles 48, 49 et donc de la position du coulisseau 35, la nature de cette variation dépendant du rapport des diamètres des poulies crantées 54-55 et 56-57.

Ce résultat est notamment dû au fait que les moyens d'entraînement en rotation de l'élément transducteur 43 en fonction de la position angulaire d'au moins l'une des bielles (48-49) fait intervenir au moins :
- une première poulie (54, 55) solidaire de la bielle (48, 49) et montée coaxialement à l'axe de pivotement de la bielle (48, 49) sur la pièce de support (39),
- une deuxième poulie (56, 57) solidaire de l'élément transducteur et montée coaxialement à l'axe de pivotement de l'élément transducteur sur la pièce de support (39),
- une courroie d'entraînement (58, 59) passant sur les deux poulies (55, 57-54, 56).

En fait, dans l'exemple illustré sur les figures 3 et 4, le mécanisme d'entraînement comprend :
- deux bielles parallèles (48, 49) montées pivotantes par l'une de leurs extrémités sur une structure (25) solidaire du corps du moteur en deux emplacements diamétralement opposés et par leurs autres extrémités sur la susdite pièce de support (39) autour d'un axe transversal commun (47),
- deux poulies primaires (54, 55) respectivement solidaires desdites bielles (48, 49) et montées coaxialement audit axe transversal commun (47),
- deux poulies secondaires (56, 57) solidaires dudit élément transducteur et montées coaxialement à l'axe de pivotement de l'élément transducteur, ces deux poulies secondaires (56, 57) constituant avec les deux poulies primaires (54, 55) deux couples de poulies en regard (55, 57-54, 56) et deux courroies d'entraînement (58, 59) passant respectivement autour des deux couples de poulies en regard (55, 57-54, 56).

Il apparaît clairement à l'examen des figures 3 et 4 qu'un avantage important de la solution précédemment décrite résulte de sa compacité et de son aptitude à la miniaturisation.

Bien entendu, l'invention ne se limite pas au mode d'exécution précédemment décrit. Ainsi, par exemple, si dans une réalisation particulière le transducteur se trouve fixé sur l'axe de rotation 50-51, le mouvement obtenu sera de type sectoriel dont l'angle sera fonction de la longueur des bielles 48-49.

## Revendications

1. Mécanisme d'entraînement utilisable dans un dispositif de balayage, ce mécanisme comprenant un dispositif de conversion d'un mouvement de rotation engendré par un organe moteur en un mouvement rectiligne alternatif, ce dispositif de conversion faisant intervenir un organe tournant planétaire (10) entraîné en rotation par l'arbre de sortie (9) de l'organe moteur (8), un pignon satellite (15) monté pivotant sur l'organe planétaire (10) et venant engrener avec une couronne à alésage dentelé (14) coaxiale audit arbre (9) et un organe d'entraînement (19) d'un élément transducteur (5) porté par un support (18) solidaire du pignon (15),
**caractérisé en ce que** :
- la couronne dentée est portée par un manchon tubulaire solidaire du corps du moteur,
- l'organe d'entraînement comprend des moyens de couplage magnétiques (23, 24) agissant sur des moyens correspondants assurant l'entraînement du transducteur,
- l'élément transducteur et l'organe d'entraînement sont disposés dans deux compartiments séparés par une cloison étanche au travers de laquelle s'effectue le couplage.

2. Mécanisme d'entraînement selon la revendication 1,
**caractérisé en ce que** le diamètre du pignon (15) est égal à la moitié du diamètre de l'alésage dentelé (14) et l'organe d'entraînement est disposé de manière à ce que, lors de la rotation de l'organe planétaire (10), ledit organe (19) effectue une trajectoire rectiligne reliant deux points diamétralement opposés de la couronne.

3. Mécanisme d'entraînement selon la revendication 1, servant à déplacer un élément transducteur (5) selon un trajet linéaire,
**caractérisé en ce que** l'organe d'entraînement (19) est couplé à une pièce de support (6) du transducteur (5) guidée selon un trajet linéaire.

4. Mécanisme d'entraînement selon la revendication 3,
**caractérisé en ce que** le couplage entre la pièce de support (6) et l'organe d'entraînement (19) s'effectue en prise directe par un moyen de couplage tel qu'un doigt d'entraînement (19) ou sans contact par un moyen de couplage tel que des moyens magnétiques (23, 24).

5. Mécanisme d'entraînement selon l'une des revendications précédentes,
**caractérisé en ce que** l'organe planétaire consiste en une pièce d'entraînement cylindrique (10) montée rotative coaxialement à l'arbre de sortie (9) du moteur par l'intermédiaire d'au moins un palier porté par un manchon tubulaire (13) solidaire du corps du moteur, ce manchon tubulaire comportant une denture interne constituant la susdite couronne dentée.

6. Mécanisme d'entraînement selon la revendication 1,
**caractérisé en ce que** le susdit organe d'entraînement est couplé à des moyens de conversion mouvement rectiligne/mouvement arciforme.

7. Mécanisme d'entraînement selon la revendication 6,
**caractérisé en ce que** les susdits moyens de conversion font intervenir :
- un coulisseau (35) déplacé par l'organe d'entraînement (34) de manière à réaliser un trajet linéaire dans un plan perpendiculaire à l'axe du moteur (26),
- une pièce de support (39) axéne parallèlement audit axe et montée axialement coulissante sur le coulisseau (35), et
- au moins une bielle (48-49) dont une extrémité est montée pivotante sur une structure (52) solidaire du corps du moteur autour d'un axe de rotation situé dans un plan axial perpendiculaire au trajet du coulisseau (35) et dont l'autre extrémité est montée pivotante sur la pièce support (39),
le déplacement arciforme d'un point de la pièce de support (39) résultant du produit de son coulissement sous l'action de la bielle (48-49) et de sa translation engendrée par le coulisseau (35).

8. Mécanisme d'entraînement selon la revendication 7, utilisé dans un dispositif de balayage d'une sonde comprenant un élément transducteur porté par une pièce de support (43) mobile selon un trajet arciforme,
**caractérisé en ce que** ledit élément transducteur est monté rotatif sur ladite pièce de support, et **en ce qu'**il comprend des moyens d'entraînement en rotation dudit élément transducteur (43) en fonction de la position angulaire de la susdite bielle (48-49).

9. Mécanisme d'entraînement selon la revendication 8,
**caractérisé en ce que** les susdits moyens d'entraînement font intervenir au moins :
- une première poulie (54, 55) solidaire de la bielle (48, 49) et montée coaxialement à l'axe de pivotement de la bielle (48, 49) sur la pièce de support (39),
- une deuxième poulie (56, 57) solidaire de l'élément transducteur et montée coaxialement à l'axe de pivotement de l'élément transducteur sur la pièce de support (39),
- une courroie d'entraînement (58, 59) passant sur les deux poulies (55, 57-54, 56).

10. Mécanisme d'entraînement selon l'une des revendications précédentes,
**caractérisé en ce qu'**il comprend :
- deux bielles parallèles (48, 49) montées pivotantes par l'une de leurs extrémités sur une structure (25) solidaire du corps du moteur en deux emplacements diamétralement opposés et par leurs autres extrémités sur la susdite pièce de support (39) autour d'un axe transversal commun (47),
- deux poulies primaires (54, 55) respectivement solidaires desdites bielles (48, 49) et montées coaxialement audit axe transversal commun (47),
deux poulies secondaires (56, 57) solidaires dudit élément transducteur et montées coaxialement à l'axe de pivotement de l'élément transducteur, ces deux poulies secondaires (56, 57) constituant avec les deux poulies primaires (54, 55) deux couples de poulies en regard (55, 57-54, 56) et deux courroies d'entraînement (58, 59) passant respectivement autour des deux

## Claims

1. A drive mechanism which can be used in a scanning device, comprising a device for converting a rotary movement generated by a motor member into an alternating rectilinear movement, this conversion device involving a planetary rotating member (10) driven into rotation by the output shaft (9) of the motor member (8), a satellite pinion (15) pivotally mounted on the planetary member (10) and meshing with a ring gear with a serrated bore (14) coaxial with said shaft (9) and a member (19) for driving a transducer element (5) borne by a support (18) integral with the pinion (15), **characterized in that**:
- the ring gear is borne by a tubular sleeve integral with the body of the motor,
- the drive member comprises magnetic coupling means (23,24) acting on corresponding means providing the driving of the transducer,
- the transducer element and the drive member are positioned in two compartments separated by a sealed partition through which the coupling is effected.

2. The drive mechanism according to claim 1,
**characterized in that** the diameter of the pinion (15) is equal to the half of the diameter of the serrated bore (14) and the drive member is positioned so that, during the rotation of the planetary member (10), said member (19) makes a rectilinear trajectory connecting two diametrically opposite points of the ring gear.

3. The drive mechanism according to claim 1, used for displacing a transducer element (5) along a linear path,
**characterized in that** the drive member (19) is coupled with a supporting part (6) of the transducer (5) guided along a linear path.

4. The drive mechanism according to claim 3,
**characterized in that** the coupling between the supporting part (6) and the drive member (19) is effected with direct meshing by a coupling means such as a drive finger (19) or without any contact by a coupling means such as magnetic means (23,24).

5. The drive mechanism according to any of the preceding claims,
**characterized in that** the planetary member consists in a cylindrical drive part (10) rotatably mounted coaxially with the output shaft (9) of the motor via at least one bearing borne by the tubular sleeve (13) integral with the body of the motor, this tubular sleeve including internal teeth forming the aforesaid ring gear.

6. The drive mechanism according to claim 1,
**characterized in that** the aforesaid drive member is coupled with rectilinear movement/arciform movement conversion means.

7. The drive mechanism according to claim 6,
**characterized in that** the aforesaid conversion means involve:
- a slide (35) displaced by the drive member (34) so as to make a linear path in a plane perpendicular to the axis of the motor (26),
- a supporting part (39) centered parallel to said axis and slidably mounted axially on the slide (35), and
- at least one connecting rod (48-49), one end of which is pivotally mounted on a structure (52) integral with the body of the motor around an axis of rotation located in an axial plane perpendicular to the path of the slide (35) and the other end of which is pivotally mounted on the supporting part,
the arciform displacement of a point of the supporting part (39) resulting from the product of its sliding under the action of the connecting rod (48-49) and of its translation generated by the slide (35).

8. The drive mechanism according to claim 7, used in a scanning device of a probe comprising a transducer element borne by a supporting part (43) mobile along an arciform path,
**characterized in that** said transducer element (43) according to the angular position of the aforesaid connecting rod (48-49).

9. The drive mechanism according to claim 8, **characterized in that** the aforesaid drive means involves at least:
- a first pulley (54, 55) integral with the connecting rod (48, 49) and mounted coaxially with the pivot axis of the connecting rod (48, 49) on the supporting part (39),
- a second pulley (56, 57) integral with the transducer element and mounted coaxially with the pivot axis of the transducer element on the supporting part (39),
- a drive belt (58, 59) passing over both pulleys (55, 57-54, 56).

10. The drive mechanism according to any of the preceding claims, **characterized in that** it comprises:
- two parallel connecting rods (48, 49) pivotally mounted through one of their ends on a structure (25) integral with the body of the motor in two diametrically opposite locations and through their other ends on the aforesaid supporting part (39) around a common transverse axis (47),
- two primary pulleys (54, 55) integral with said connecting rods (48, 49) respectively, and mounted coaxially with said common transverse axis (47),
- two secondary pulleys (56, 57) integral with said transducer element and mounted coaxially with the pivot axis of the transducer element, both of these secondary pulleys (56, 57) forming with both primary pulleys (54, 55) two facing pairs of pulleys (55, 57-54, 56) and two drive belts (58, 59) passing around both facing pairs of pulleys (55, 57-54, 56), respectively.

## Patentansprüche

1. Antriebsmechanismus, der in einem Abtastgerät verwendbar ist, wobei dieser Mechanismus eine Vorrichtung zur Umsetzung einer durch einen Motor erzeugten Rotationsbewegung in eine alternative geradlinige Bewegung umfasst, wobei diese Umsetzvorrichtung ein Planeten-Drehorgan (10) intervenieren lässt, das unter Rotation durch die Abtriebswelle (9) des Motororgans (8) angetrieben wird, und ein Satellitenrad (15), das auf dem Planetenorgan (10) drehbar montiert ist und das mit einer mit gezahnter Bohrung versehenen Krone (14), die koaxial an der genannten Welle (9) liegt, in Eingriff kommt und ein Antriebsorgan (19) eines Umformerelements (5), das von einem kraftschlüssig mit dem Ritzel (15) verbundenen Träger (18) getragen wird, **gekennzeichnet dadurch, dass:**
- die gezahnte Krone von einer rohrförmigen, kraftschlüssig mit dem Motorkörper verbundenen Muffe gehalten ist,
- das Antriebsorgan magnetische Kopplungselemente (23, 24) umfasst, die auf die entsprechenden Elemente einwirken und so den Antrieb des Umformers sicherstellen,
- das Umformerelement und das Antriebsorgan in zwei Abteilungen angeordnet sind, die durch eine dichte Scheidewand getrennt sind, durch welche die Kopplung stattfindet.

2. Antriebsmechanismus nach Anspruch 1, **gekennzeichnet dadurch, dass** der Durchmesser des Ritzels (15) gleich die Hälfte des Durchmessers der gezahnten Bohrung (14) ist und das Antriebsorgan so angeordnet ist, dass besagtes Organ (19) bei der Rotation des Planeten-Organs (10) eine geradlinige Strecke vollzieht, indem es zwei diametral gegenüberliegende Punkte der Krone verbindet.

3. Antriebsmechanismus nach Anspruch 1, der dazu dient, um ein Umformerelement (5) gemäß einer linearen Strecke zu verschieben, **gekennzeichnet dadurch, dass** das Antriebsorgan (19) an einem Trägerteil (6) des Umformers (5) gekoppelt ist, der gemäß einer linearen Strecke geführt wird.

4. Antriebsmechanismus nach Anspruch 3, **gekennzeichnet dadurch, dass** die Kopplung zwischen dem Trägerteil (6) und dem Antriebsorgan (19) durch einen direkten Griff über ein Koppelelement wie ein Antriebsfinger (19) oder kontaktlos über ein Koppelelement wie Magnetelemente (23, 24) erfolgt.

5. Antriebsmechanismus nach einem der vorangehenden Ansprüche, **gekennzeichnet dadurch, dass** das Planetenorgan aus einem zylindrischen Antriebsteil (10) besteht, das koaxial drehbar an der Abtriebswelle (9) des Motors angebracht ist, mittels mindestens einem Lager, das von einer rohrförmigen, kraftschlüssig mit dem Motorkörper verbundenen Muffe (13) gehalten wird, wobei diese rohrförmige Muffe mit einer inneren Verzahnung versehen ist und so die besagte gezahnte Krone bildet.

6. Antriebsmechanismus nach Anspruch 1, **gekennzeichnet dadurch, dass** besagtes Antriebsorgan an Umformerelemente für geradlinige Bewegung/bogenförmige Bewegung gekoppelt ist.

7. Antriebsmechanismus nach Anspruch 6, **gekennzeichnet dadurch, dass** die besagten Umformerelemente folgendes intervenieren lassen:
- einen Schieber (35), der von dem Antriebsorgan (34) verschoben wird, um in einer senkrechten Ebene zur Achse des Motors (26) eine lineare Strecke zu vollziehen,
- ein parallel an die genannte Achse angelenktes und axial gleitend auf dem Schieber (35) angebrachtes Trägerteil (39), und
- mindestens eine Pleuelstange (48-49), wobei ein Ende auf einer kraftschlüssig mit dem Motorkörper verbundenen Struktur (52) drehbar um eine Drehachse angebracht ist, die in einer axialen Ebene lotrecht zur Strecke des Schiebers (35) liegt und deren anderes Ende drehbar auf dem Trägerteil (39) angebracht ist,
wobei sich die bogenförmige Verschiebung einer Stelle des Trägerteils (39) aus dessen Verschiebung unter der Einwirkung der Pleuelstange (48-49) und aus seiner durch den Schieber (35) erzeugten Translation ergibt.

8. Antriebsmechanismus nach Anspruch 7, der in einem Abtastgerät einer Sonde benutzt wird, das ein von einem Trägerteil getragenes Umformerelement (43) umfasst, das gemäß einer bogenförmigen Strecke beweglich ist, **gekennzeichnet dadurch, dass** besagtes Umformerelement drehbar auf dem Trägerteil montiert ist, **und dass** es entsprechend der Winkelposition der besagten Pleuelstange (48, 49) rotierende Antriebselemente des besagten Umformerelements (43) umfasst.

9. Antriebsmechanismus nach Anspruch 8, **gekennzeichnet dadurch, dass** die besagten Antriebselemente mindestens folgendes intervenieren lassen:
- eine erste Riemenscheibe (54, 55), die kraftschlüssig mit der Pleuelstange (48, 49) verbunden und koaxial an der Drehachse der Pleuelstange (48, 49) auf dem Trägerteil (39) angebracht ist,
- eine zweite Riemenscheibe (56, 57), die kraftschlüssig mit dem Umformerelement verbunden und koaxial an der Drehachse des Umformerelements auf dem Trägerteil (39) angebracht ist,
- einen Antriebsriemen (58, 59), der auf den beiden Riemenscheiben (55, 57 - 54, 56) liegt.

10. Antriebsmechanismus nach einem der vorangehenden Ansprüche, **gekennzeichnet dadurch, dass** dieser folgendes umfasst:
- zwei parallele Pleuelstangen (48, 49), die über eines seiner Enden 9 drehbar auf eine kraftschlüssig mit dem Motorkörper verbundene Struktur (25) an zwei gegenüberliegenden Stellen und über deren andere Enden auf dem besagten Trägerteil (39) um eine gemeinsame Querachse (47) herum angebracht sind,
- zwei primäre, jeweils kraftschlüssig mit den besagten Pleuelstangen (48, 49) verbundene und koaxial an der genannten gemeinsamen Querachse (47) angebrachte Riemenscheiben (54, 55),
- zwei sekundäre, kraftschlüssig mit dem genannten Umformerelement verbundene Riemenscheiben (56, 57), die koaxial zur Drehachse des Umformerelements angebracht sind,
wobei beide sekundäre Riemenscheiben (56, 57) mit den beiden primären Riemenscheiben (54, 55) zwei Paare von einander gegenüberliegenden Riemenscheiben (55, 57 - 54, 56) und zwei Antriebsriemen (58, 59) bilden, die jeweils um zwei Paare von einander gegenüberliegenden Riemenscheiben (55, 54 - 57, 56) herumführen.
